# EUROPEAN PATENT APPLICATION

(11) **EP 2 752 166 A2**
(43) Date of publication of application: **09.07.2014**
(21) Application number: 12827004.8
(22) Date of filing: 29.08.2012
(51) Int. Cl.: A61B 17/29, A61B 17/00

(54) **MINIMALLY INVASIVE SURGICAL INSTRUMENT HAVING AN IMPROVED JOINT**

(30) Priority: 29.08.2011 KR 20110086738
(71) Applicant: Movasu, Inc., Seoul 110-450 (KR)
(72) Inventor: JEONG, Chang Wook, Seoul 110-450 (KR); SIN, Chun Chol, Seoul 110-450 (KR); KIM, Sung Ryul, Seoul 110-450 (KR); KIM, Hyung Tae, Seoul 110-450 (KR)
(74) Representative: Zardi, Marco
(86) International application number: PCT/KR2012/006908
(87) International publication number: WO 2013/032229

(57) **Abstract**

The present invention relates to a minimally invasive surgical instrument having an improved joint. According to one aspect of the present invention, provided is a minimally invasive surgical instrument comprising a shaft, a joint part which is coupled to one end of the shaft, and an end effector which is coupled to the joint part and can effect joint movement thereby. The joint part comprises a plurality of joint links, and a joint for pitch-direction operation and a joint for yaw direction operation, the joints being formed by interposing at least one of the plurality of joint links.

## Description

### FIELD OF THE INVENTION

The present invention relates to a minimally invasive surgical instrument having an improved joint unit.

### BACKGROUND

Minimally invasive surgery is a surgical approach that involves the use of instruments inserted through several tiny incision openings to perform a surgery causing minimal tissue trauma in human or animal bodies.

The minimally invasive surgery relatively reduces changes in metabolism of a patient in the period of post-surgical care, so it facilitates rapid recovery of the patient. Therefore, the minimally invasive surgery shortens the length of hospitalization of the patient after the surgery and allows the patient to return to normal physical activities in a short period of time. In addition, the minimally invasive surgery causes less pain and leaves fewer scars on the patient's body after the surgery.

One of the general forms of the minimally invasive surgery is endoscopy. Among the others, a laparoscopy that involves minimally invasive inspection and operation inside abdominal cavity is known as the most general form of endoscopy. To operate a standard laparoscopic surgery, the abdomen of the patient is insufflated with gas and at least one small incision is formed to provide an entrance for laparoscopic surgical instruments, through which a trocar is inserted. When performing the surgery, it is general that a user puts the laparoscopic surgical instruments into a surgical site or the like through the trocar, and manipulates (or controls) the instruments from the outside of abdominal cavity. In general, the laparoscopic surgical instruments include a laparoscope (for observation of a surgical site) and other working tools. Herein, the working tools are similar to the conventional tools used for small incision surgery, except that the end effector or working end of each tool is separated from its handle or the like by a shaft. For instance, the working tools may include a clamp, a grasper, scissors, a stapler, a needle holder, and so forth. Meanwhile, the user monitors the procedure of the surgery through a monitor that displays the images of the surgical site which are taken by the laparoscope. The endoscopic approaches similar to the above are broadly used in retroperitoneoscopy, pelviscopy, arthroscopy, cisternoscopy, sinuscopy, hysteroscopy, nephroscopy, cystoscopy, urethroscopy, pyeloscopy, and so on.

The inventor(s) has developed various minimally invasive surgical instruments useful for the above-mentioned minimally invasive surgeries and has already disclosed the features of the structures and effects of the same in Korean Patent Application Nos. 2008-51248, 2008-61894, 2008-79126 and 2008-90560, the contents of which are incorporated herein by reference in its entirety. Additionally, the inventor(s) have also introduced a minimally invasive surgical instrument with improved functionality, which is more advantageous for users and patients, in Korean Patent Application Nos. 2010-115152, 2011-3192, 2011-26243 and 2011-29771, the contents of which are incorporated herein by reference in its entirety.

Herein, the inventor(s) now present a minimally invasive surgical instrument having a more improved joint unit than those disclosed in the above Korean applications or others.

### SUMMARY OF THE INVENTION

One object of the present invention is to provide a minimally invasive surgical instrument to allow an end effector to smoothly carry out rolling motion.

Another object of this invention is to provide a minimally invasive surgical instrument to allow an end effector to smoothly carry out rolling motion when it has carried out joint motion or even when the state of the joint motion has been fixed.

According to one aspect of the invention to achieve the objects as described above, there is provided a minimally invasive surgical instrument comprising a shaft; a joint unit being connected to one end of the shaft; and an end effector being connected to the joint unit and capable of carrying out joint motion thereby, wherein the joint unit comprises a plurality of joint links; and joints for pitch direction operation and joints for yaw direction operation being formed with at least one of the plurality of joint links being interposed therebetween, and wherein a passage for a torque transmission member is formed in each of the plurality of joint links.

In addition, there may be provided other configurations to implement this invention.

According to the invention, there is provided a minimally invasive surgical instrument to allow an end effector to smoothly carry out rolling motion.

According to the invention, there is provided a minimally invasive surgical instrument to allow an end effector to smoothly carry out rolling motion when it has carried out joint motion or even when the state of the joint motion has been fixed.

### BRIEF DESCRIPTION OF THE DRAWINGS

Fig. 1 shows the overall appearance of a minimally invasive surgical instrument according to one embodiment of the invention.
Fig. 2 is a partially enlarged view of a joint unit 300 of the minimally invasive surgical instrument shown in Fig. 1.
Fig. 3 is a perspective view of some components such as an end effector 200 and a joint unit 300 of a minimally invasive surgical instrument according to one embodiment of the invention.
Figs. 4 and 5 are exploded perspective views of the components of the minimally invasive surgical instrument shown in Fig. 3.
Fig. 6 is a cross-sectional view to show in more detail how the various components of the minimally invasive surgical instrument shown in Fig. 4 are connected.

### DETAILED DESCRIPTION OF THE PREFERRED EMBODIMENTS

In the following detailed description of the invention, references are made to the accompanying drawings that show, by way of illustration, specific embodiments in which the invention may be practiced. These embodiments are described in sufficient detail to enable those skilled in the art to practice the invention. It is to be understood that the various embodiments of the invention, although different from each other, are not necessarily mutually exclusive. For example, specific shapes, structures, or characteristics described herein may be implemented as modified from one embodiment to another embodiment without departing from the spirit and the scope of the invention. Furthermore, it shall be understood that the locations or arrangements of individual elements within each embodiment may be also modified without departing from the spirit and the scope of the invention. Therefore, the following detailed description is not to be taken in a limiting sense, and the scope of the invention is to be taken as encompassing the scope of the appended claims and all equivalents thereof. In the drawings, like reference numerals refer to the same or similar elements throughout the several views.

Hereinafter, preferred embodiments of the present invention will be described in detail with reference to the accompanying drawings to enable those skilled in the art to easily implement the invention.

Meanwhile, it should be understood that the term "connection" herein encompasses a direct connection or an indirect connection (i.e., via separate components) between mechanical or other types of components.

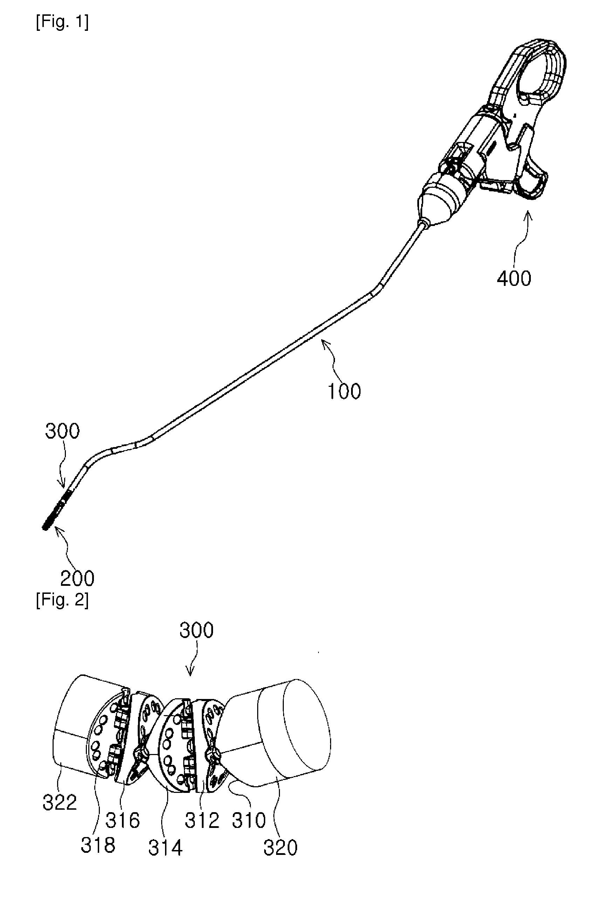

Fig. 1 shows the overall appearance of a minimally invasive surgical instrument according to one embodiment of the invention.

Reference will be made to Fig. 1. The minimally invasive surgical instrument may comprise a shaft 100; an end effector 200 being connected to one end of the shaft 100 to perform surgery by using surgical tools (not shown) or functioning itself as a surgical tool; a joint unit 300 to connect the shaft 100 and the end effector 200 and to provide the end effector 200 with joint functionality; and a handling unit 400 being connected to the other end of the shaft 100 and capable of being held and manipulated by a user.

First, the shaft 100 may include a cavity therein to support and pass at least one wire or torque transmission member, in the same manner as those of the minimally invasive surgical instruments disclosed in the aforementioned Korean patent applications. (The torque transmission member is mainly intended for the rolling motion of the end effector 200, while the shaft 100 may function itself as the torque transmission member in some cases.) The shaft 100 may comprise at least one segment as necessary. Further, the shaft 100 may comprise a bend in at least a part thereof.

Next, the end effector 200 may carry out joint motion, rolling motion, opening/closing motion and the like by the action of the at least one wire or torque transmission member passing from the handling unit 400 to the joint unit 300 via the shaft 100, in the same manner as those of the minimally invasive surgical instruments disclosed in the aforementioned Korean patent applications. The tip of the end effector 200 may be implemented in the form of a clamp, a grasper, a pair of scissors, a stapler, a needle holder, a hook-type electrode or the like.

Next, the joint unit 300 may act together with the at least one wire or torque transmission member to allow the end effector 200 to carry out joint motion, rolling motion and the like, in the same manner as those of the minimally invasive surgical instruments disclosed in the aforementioned Korean patent applications.

Finally, the handling unit 400 may control the joint motion, rolling motion, opening/closing motion and the like of the end effector 200 according to the user's manipulation, in the same manner as those of the minimally invasive surgical instruments disclosed in the aforementioned Korean patent applications. To allow for such control, the at least one wire or torque transmission member may be connected to the handling unit 400.

Fig. 2 is a partially enlarged view of the joint unit 300 of the minimally invasive surgical instrument shown in Fig. 1.

Reference will be made to Fig. 2. The joint unit 300 comprises a first connecting unit 320 and a second connecting unit 322, and may further comprise a first joint link 312, a second joint link 314 and a third joint link 316 therebetween. In addition, a first joint part 310 may be formed at one end of the first connecting unit 320, and a second joint part 318 may be formed at one end of the second connecting unit 322. As shown, the first connecting unit 320, the first joint link 312, the second joint link 314, the third joint link 316 and the second connecting unit 322 may be disposed in sequence so that each joint between adjacent two of them (i.e., a joint P for pitch direction operation or a joint Y for yaw direction operation) is alternated. In connection with the principle of configuring the joints, further reference may be made to Korean Patent Application No. 2011-3192.

Further, each of the first, second and third joint links 312, 314 and 316 and the first and second connecting units 320 and 322 (or the first and second joint parts 310 and 318) may provide a penetration passage (e.g., a penetration hole) or a similar binding site for a pitch wire (not shown) or a yaw wire (not shown) in the same manner as the joint links or connecting units disclosed in Korean Patent Application No. 2011-3192. Accordingly, the end effector 200 may carry out joint motion by the action of the wires in the joint unit 300.

Furthermore, each of the first, second and third joint links 312, 314 and 316 and the first and second connecting units 320 and 322 (or the first and second joint parts 310 and 318) may have a rotating link member or a slope similar to those disclosed in Korean Patent Application No. 2011-3192. Thus, adjacent two of the first connecting unit 320, the first joint link 312, the second joint link 314, the third joint link 316 and the second connecting unit 322 may be connected by means of the rotating link member to form an individual joint that operates within a predetermined range.

Meanwhile, although it has been described above that the joint unit 300 may be configured such that the joint P for pitch direction operation and the joint Y for yaw direction operation are disposed therein in the order of P-Y-P-Y or Y-P-Y-P, it shall be understood that those skilled in the art may also configure the joint unit 300 to have a more extended joint structure such as P-Y-P-Y-P-Y or Y-P-Y-P-Y-P in order to implement more delicate joint motion.

The configuration of the joint unit 300 according to the invention will be further described below.

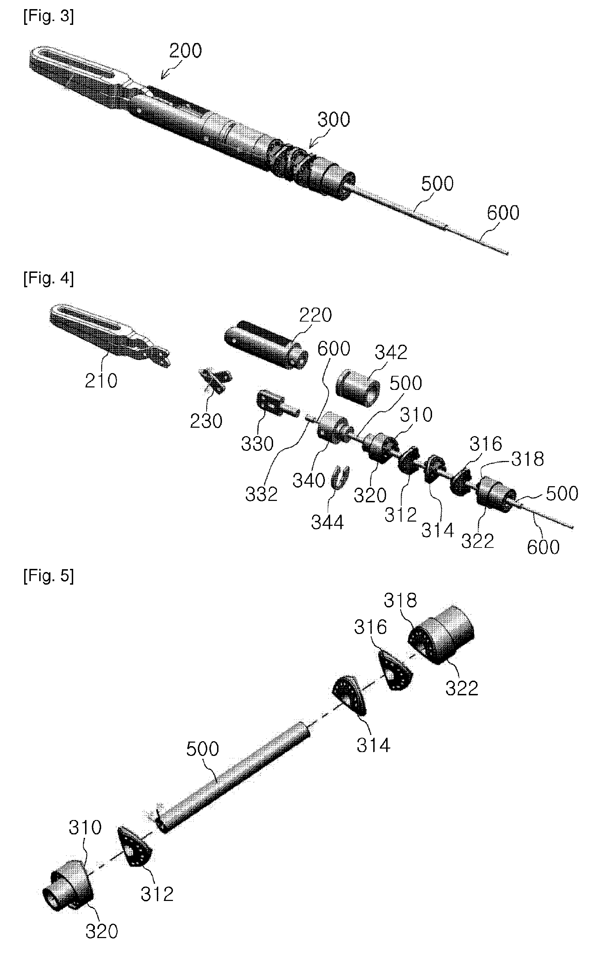

Fig. 3 is a perspective view of some components such as the end effector 200 and the joint unit 300 of the minimally invasive surgical instrument according to one embodiment of the invention. Further, Figs. 4 and 5 are exploded perspective views of the components of the minimally invasive surgical instrument shown in Fig. 3.

Reference will be made to Fig. 3. As described above, the end effector 200 and the joint unit 300 may be connected to each other, and a torque transmission member 500 or an opening/closing wire 600 may be supported and passed through some part of the joint unit 300 (preferably the longitudinal central axis thereof) toward the end effector 200. Further, the opening/closing wire 600 may be supported and passed through a cavity or passage within the torque transmission member 500. In general, the basic configuration or function of the torque transmission member 500 or the opening/closing wire 600 is as described above. Meanwhile, particular reference may be made to Korean Patent Application No. 2011-29771 in connection with various kinds of the torque transmission member 500 which may be supported and passed within the bended or bendable components such as the shaft 100 and the joint unit 300.

Reference will be made to Figs. 4 and 5.

First, the end effector 200 may comprise a working end 210, a working end connecting unit 220 and an X-shaped link 230. The configuration and operating principle of the end effector 200 may be similar to those of the end effectors disclosed in the aforementioned Korean patent applications.

Further, the first connecting unit 320, the first joint link 312, the second joint link 314, the third joint link 316 and the second connecting unit 322 may be connected in sequence in the joint unit 300 as described above. A passage for the torque transmission member 500 or the opening/closing wire 600 may be provided, preferably centrally, in each of the first connecting unit 320, the first joint link 312, the second joint link 314, the third joint link 316 and the second connecting unit 322. In particular, the passage may be configured such that the torque transmission member 500 may carry out rolling motion without being restricted by any of the first connecting unit 320, the first joint link 312, the second joint link 314, the third joint link 316 and the second connecting unit 322. The above configuration enables the torque transmission member 500 to smoothly cause the rolling motion of the end effector 200 without affecting the joint motion thereof. In this case, the coordination between the shaft and the joint unit for the rolling motion of the end effector is not necessary, which has been required in the minimally invasive surgical instruments of the aforementioned Korean patent applications.

Further, the joint unit 300 may further comprise an opening/closing wire fixing unit 330, an opening/closing wire cap 332, a rolling connecting unit 340, a holder 342 and a fastener 344. (Herein, all of those are deemed to be the components of the joint unit 300 for convenience, while some of those may be considered to belong to the end effector 200 in some point of view.)

First, one end of the opening/closing wire 600 may be fixed to the opening/closing wire fixing unit 330 via the opening/closing wire cap 332 as the opening/closing wire 600 has been extended out of the torque transmission member 500. Thus, when the opening/closing wire 600 is pulled, the opening/closing wire fixing unit 330 may act on the X-shaped link 230 connected thereto so that the working end 210 is closed. (Naturally, the configuration of the end effector 200 may be slightly changed so that the end effector 200 is opened when the opening/closing wire 600 is pulled.)

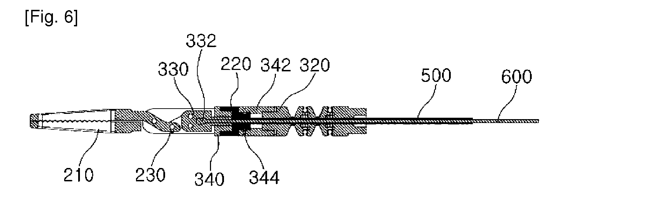

Further, the rolling connecting unit 340 may be connected to the torque transmission member 500 to rotate as the torque transmission member 500 carries out rolling motion. This rotation may cause the rotation of the working end connecting unit 220, which may be coupled to the rolling connecting unit 340 by means of the holder 342 as shown in Fig. 6, and further cause the rotation of the working end 210 connected thereto (i.e., the rolling motion of the end effector 200). (Fig. 6 is a cross-sectional view to show in more detail how the various components of the minimally invasive surgical instrument shown in Fig. 4 are connected.) In order to easily tighten or loosen the above coupling rendered by the holder 342, the fastener 344 which may be fitted in the connecting part between the working end connecting unit 220 and the holder 342 may be further employed as necessary.

### Applications

According to an application of the present invention, those skilled in the art may partially change the form and such of the handling unit or the like so that the wire or torque transmission member of the minimally invasive surgical instrument may be operated by an electric motor or the like of another motor-based system (not shown) such as a surgical robot, as necessary.

Although the present invention has been described in terms of specific items such as detailed elements as well as the limited embodiments and the drawings, they are only provided to help general understanding of the invention, and the present invention is not limited to the above embodiments. It will be appreciated by a person of ordinary skill in the art that various modifications and changes may be made from the above description.

Therefore, the spirit of the present invention shall not be limited to the above-described embodiments, and the entire scope of the appended claims and their equivalents will fall within the scope and spirit of the invention.

## Claims

1. A minimally invasive surgical instrument comprising:
a shaft;
a joint unit being connected to one end of the shaft; and
an end effector being connected to the joint unit and capable of carrying out joint motion thereby,
wherein the joint unit comprises:
a plurality of joint links; and
joints for pitch direction operation and joints for yaw direction operation being formed with at least one of the plurality of joint links being interposed therebetween, and
wherein a passage for a torque transmission member is formed in each of the plurality of joint links.

2. A minimally invasive surgical instrument as claimed in Claim 1, wherein a penetration hole for a joint motion wire is formed in each of the plurality of joint links.

3. A minimally invasive surgical instrument as claimed in Claim 1, wherein at least one of the plurality of joint links comprises a rotating link member at one side thereof and another rotating link member at the other side thereof, the another rotating link member being disposed substantially perpendicular to the direction in which the rotating link member is disposed.

4. A minimally invasive surgical instrument as claimed in Claim 1, wherein at least one of the plurality of joint links has a slope for the joint motion.

5. A minimally invasive surgical instrument as claimed in Claim 1, wherein in the joint unit, there are provided at least two joints for pitch direction operation and at least two joints for yaw direction operation, and the at least four joints are disposed such that the joints for pitch direction operation and the joints for yaw direction operation are alternated.

6. A minimally invasive surgical instrument as claimed in Claim 1, wherein the joint unit further comprises a connecting unit for connection with the end effector, and the passage for the torque transmission member is formed in the connecting unit.

7. A minimally invasive surgical instrument as claimed in Claim 1, wherein the joint unit further comprises a rolling connecting unit, and the rolling connecting unit is connected to the torque transmission member to rotate as the torque transmission member carries out rolling motion.

8. A minimally invasive surgical instrument as claimed in Claim 7, wherein the end effector and the rolling connecting unit are removably coupled to each other.

9. A minimally invasive surgical instrument as claimed in Claim 7, wherein the end effector and the rolling connecting unit are coupled to each other by means of a holder and a fastener.

10. A minimally invasive surgical instrument as claimed in Claim 1, further comprising an opening/closing wire to open or close the end effector, the opening closing wire being supported and passed within the torque transmission member.
